# EUROPEAN PATENT SPECIFICATION

(11) **EP 3 976 835 B1**
(45) Date of publication and mention of the grant of the patent: **17.07.2024**
(21) Application number: 20731522.7
(22) Date of filing: 29.05.2020
(51) Int. Cl.: C12Q 1/6895

(54) **A METHOD FOR DETERMINING A YEAST HAVING EXTRACELLULAR GLUCOAMYLASE STA1P ACTIVITY IN A SAMPLE AND TOOLS AND USES RELATED THERETO**
VERFAHREN ZUR BESTIMMUNG EINER HEFE MIT EXTRAZELLULÄRER GLUCOAMYLASE-STA1P-AKTIVITÄT IN EINER PROBE SOWIE WERKZEUGE UND VERWENDUNGEN IN ZUSAMMENHANG DAMIT
PROCÉDÉ DE DÉTERMINATION D'UNE LEVURE AYANT UNE ACTIVITÉ DE GLUCOAMYLASE STA1P EXTRACELLULAIRE DANS UN ÉCHANTILLON, ET OUTILS ET UTILISATIONS ASSOCIÉES

(30) Priority: 31.05.2019 FI 20195454
(43) Date of publication of application: 06.04.2022
(73) Proprietor: Teknologian Tutkimuskeskus VTT OY, 02150 Espoo (FI)
(72) Inventor: KROGERUS, Kristoffer, 02044 VTT (FI); GIBSON, Brian, 02044 VTT (FI)
(74) Representative: Berggren Oy
(86) International application number: PCT/FI2020/050360
(87) International publication number: WO 2020/240091

(56) References cited:
- JP-A- 2011 223 940
- US-A1- 2006 046 253
- US-A1- 2007 118 916
- "Annex 1 (sequence alignments)", 14 July 2020, article ANONYMOUS: "Annex 1 (sequence alignments)", pages: 1 - 2, XP055714636
- HIROMASA YAMAUCHI ET AL: "Rapid Methods for Detecting Saccharomyces Diastaticus , a Beer Spoilage Yeast, Using the Polymerase Chain Reaction", JOURNAL OF THE AMERICAN SOCIETY OF BREWING CHEMISTS., vol. 56, no. 2, 1 April 1998 (1998-04-01), US, pages 58 - 63, XP055714514, ISSN: 0361-0470, DOI: 10.1094/ASBCJ-56-0058
- KEUN KIM ET AL: "Cloning of a new allelic variant of aglucoamylase gene and its introduction into industrial yeasts", APPLIED BIOCHEMISTRY AND BIOTECHNOLOGY ; PART A: ENZYME ENGINEERING AND BIOTECHNOLOGY, HUMANA PRESS INC, NEW YORK, vol. 44, no. 2, 1 February 1994 (1994-02-01), pages 161 - 185, XP035176922, ISSN: 1559-0291, DOI: 10.1007/BF02921653
- GEORGE BAJSZÁR ET AL: "Properties and engineering of a mutantpromoter of", APPLIED BIOCHEMISTRY AND BIOTECHNOLOGY ; PART A: ENZYME ENGINEERING AND BIOTECHNOLOGY, HUMANA PRESS INC, NEW YORK, vol. 44, no. 2, 1 February 1994 (1994-02-01), pages 187 - 204, XP035176924, ISSN: 1559-0291, DOI: 10.1007/BF02921654
- KROGERUS KRISTOFFER ET AL: "A re-evaluation of diastaticstrains and their role in brewing", APPLIED MICROBIOLOGY AND BIOTECHNOLOGY, SPRINGER BERLIN HEIDELBERG, BERLIN/HEIDELBERG, vol. 104, no. 9, 13 March 2020 (2020-03-13), pages 3745 - 3756, XP037093516, ISSN: 0175-7598, [retrieved on 20200313], DOI: 10.1007/S00253-020-10531-0
- KROGERUS KRISTOFFER ET AL: "A deletion in theSTA1promoter determines maltotriose and starch utilization inSTA1+Saccharomyces cerevisiaestrains", APPLIED MICROBIOLOGY AND BIOTECHNOLOGY, SPRINGER BERLIN HEIDELBERG, BERLIN/HEIDELBERG, vol. 103, no. 18, 26 July 2019 (2019-07-26), pages 7597 - 7615, XP036875349, ISSN: 0175-7598, [retrieved on 20190726], DOI: 10.1007/S00253-019-10021-Y

## Description

### FIELD OF THE INVENTION

The present invention relates to the fields of genetic testing and furthermore determining specific yeasts. Specifically, the invention as defined in the appended claims relates to a method for determining a yeast having extracellular glucoamylase STA1p activity in a sample, a primer capable of hybridizing with *STA1* gene of a yeast, a primer pair comprising two primers capable of hybridizing with *STA1* gene of a yeast, and a probe capable of hybridizing with the promoter of *STA1* gene of a yeast e.g. within a very specific region. Also, the present invention as defined in the appended claims relates to a kit for genetic testing. And still, the present invention as defined in the appended claims relates to use of the primer, primer pair, probe or kit of the present invention for detecting the presence or absence of a deletion in the promoter of *STA1* gene in a yeast.

### BACKGROUND OF THE INVENTION

Some diastatic strains of yeast are considered spoilage microorganisms in industrial lager fermentations. These strains, including but not limited to *Saccharomyces cerevisiae* formerly known as *Saccharomyces diastaticus*, are capable of producing an extracellular glucoamylase that enables fermentation of starch and oligosaccharides. This in turn, causes super-attenuation in the beer, which leads to increased carbon dioxide and ethanol levels, possible off-flavours, and a drier mouthfeel. In the case of contamination in packaged beer, diastatic S. *cerevisiae* may even cause exploding bottles and cans, endangering the consumer.

The extracellular glucoamylase in diastatic S. *cerevisiae* is coded for by the *STA* genes (Tamaki, 1978, MGG Mol. Gen. Genet. Volume 164, issue 2, 205-209; Yamashita et al., 1985, J. Bacteriol. 161, 574-582; Yamashita, et al., 1985, J. Bacteriol. 161, 567-573). Three highly homologous *STA* genes (*STA1-3*) have been described (Tamaki, 1978, MGG Mol. Gen. Genet. Volume 164, issue 2, 205-209; Lambrechts et al. 1991, Gene, vol. 100, 95-103). STA1p is the main cause of spoilage features in said S. *cerevisiae.* Genetic differentiation of spoilage and non-spoilage yeast strains is important e.g. for the food industry in order to minimize the production costs and optimize the quality of end products.

Current detection methods for diastatic *S*. *cerevisiae* rely mainly on either the microbiological detection through culturing on specialized selective growth media, or the molecular detection of the *STA1* gene through conventional or quantitative PCR (Brandl, A., 2006, PhD thesis, Entwicklung und Optimierung von PCR-Methoden zur Detektion und Identifizierung von brauereirelevanten Mikroorganismen zur Routine-Anwendung in Brauereien, 179 pages, Technische Universität München); Meier-Dörnberg et al., 2018, FEMS Yeast Res. 18, issue 4, foy023; Van Der Aa Kühle and Jespersen, 1998, Int. J. Food Microbiol. 43(3):205-213; Yamauchi et al., 1998, J. Am. Soc. Brew. Chem. 56, 58-63).

The main weakness of the microbiological methods is that they are time-consuming and thus expensive. While the current molecular methods are rapid, they are not able to determine all those yeast strains that do not show spoilage potential. Therefore, with the current genetic tests these benign strains would be erroneously flagged as diastatic and needlessly removed. For example breweries might unnecessarily be recalling and discarding beer that has been erroneously flagged as containing diastatic yeast based on the prior art genetic tests. One example of said unreliable prior art genetic tests has been described in JP2011223940 defining a PCR based method for detecting specific microorganisms, wherein in one embodiment said method comprises detecting a target gene *STA1* in *S*. *cerevisiae.*

H!ROMASA YAMAUCHI ET AL, JOURNAL OF THE AMERICAN SOCIETY OF BREWING CHEMISTS., vol. 56, no. 2, 1 April 1998, pages 58-63 refers to rapid methods for fetecting Saccharomyces Diastaticus.

Indeed, there remains a significant unmet and long-felt need for a method and tools for specifically detecting only those yeasts, which are diastatic, or on the other hand determining exactly those yeasts, which are non-diastatic.

### BRIEF DESCRIPTION OF THE INVENTION

The present invention is defined in the appended claims. There is considerable variability in diastatic ability and beer-spoilage potential in yeast strains carrying the *STA1* gene. Now, the present invention provides a method, which is able to detect non-spoilage and spoilage yeast strains very reliably, also among those yeasts which carry a *STA1* gene. More specifically the present invention is based on the surprising finding that non-spoilage yeast strains carrying the *STA1* gene have a specific alteration in said *STA1* gene. Therefore, the objects of the invention, including but not limited to obtaining specific genetic methods and tools for differentiating spoilage and non-spoilage yeast strains, are achieved by utilizing the specific region of the *STA1* gene.

The present invention overcomes the defects and problems of the prior art including lack of methods for determining yeast strains that carry the *STA1* gene but do not show spoilage potential. Indeed, the number of false diastatic positives among yeast strains can be greatly reduced with the method and tools presented herein. The genetic method of the present invention is cheap and fast enabling the results in hours instead of days. Also, the method and tools of the present invention enable lowered production costs and effective large-scale production processes of food and beverages, because based on the present invention only the specific non-diastatic yeast strains can be utilized in said processes. Indeed, optimizing use of suitable, i.e. non-diastatic, yeast strains e.g. in food industry is possible with the present invention and therefore, unnecessary economic losses can be avoided.

More specifically it has now been surprisingly found that the poorly diastatic yeasts have a specific deletion in the promoter or upstream region of *STA1* gene. Said deletion greatly decreases diastatic ability and the expression of *STA1.* A specific object of the present invention is thus to provide tools and a method for effectively differentiating highly and poorly diastatic yeasts by utilizing the specific region of the *STA1* gene shown to be deleted in poorly diastatic yeasts.

The present invention relates to a method for determining a yeast having extracellular glucoamylase STA1p activity in a sample, wherein the method comprises detecting the presence or absence of a deletion in the promoter of *STA1* gene in a sample, as defined in the appended claims.

The present invention relates to a method for determining a yeast having extracellular glucoamylase STA1p activity in a sample, wherein the method comprises allowing a primer or probe capable of hybridizing with the promoter of *STA1* gene (e.g. within or partly within nucleotides 1127-2288 as numbered in SEQ ID NO: 1) to hybridize with a polynucleotide of the sample, and detecting the presence or absence of a deletion in the promoter of *STA1* gene in a sample.

Also, the present invention relates to a primer capable of hybridizing with *STA1* gene of a yeast (optionally for determining a yeast having extracellular glucoamylase STA1p activity or for detecting the presence or absence of a deletion in the promoter of STA1 gene), wherein said primer is capable of hybridizing with the promoter of *STA1* gene within nucleotides 1127-2288 as numbered in SEQ ID NO: 1, wherein said primer is selected from SEQ ID NO: 2, 3 or 4.

Also, the present invention relates to a primer pair (optionally for determining a yeast having extracellular glucoamylase STA1p activity or for detecting the presence or absence of a deletion in the promoter of STA1 gene) comprising two primers capable of hybridizing with *STA1* gene of a yeast, wherein at least one of said primers is capable of hybridizing with the promoter of *STA1* gene within nucleotides 1127-2288 as numbered in SEQ ID NO: 1, and wherein said primer is selected from SEQ ID NO: 2, 3 or 4.

Still, the present invention relates to a probe capable of hybridizing with the promoter of *STA1* gene of a yeast within or at least partially within nucleotides 1127-2288 as numbered in SEQ ID NO: 1 (optionally for determining a yeast having extracellular glucoamylase STA1p activity or for detecting the presence or absence of a deletion in the promoter of STA1 gene), wherein said probe is selected from SEQ ID NO: 2, 3 or 4.

And still, the present invention relates to a kit for genetic testing comprising a primer, primer pair or probe of the present invention and optionally reagents for performing said genetic testing.

Still furthermore, the present invention relates to use of the primer, primer pair, probe or kit of the present invention for detecting the presence or absence of a deletion in the promoter of *STA1* gene in a yeast.

Disclosed, but not part of the invention, is a (genetically) modified yeast comprising a deletion in the promoter of *STA1* gene, e.g. said deletion comprising or being within nucleotides 1127-2288 as numbered in SEQ ID NO: 1.

Other objects, details and advantages of the present invention will become apparent from the following drawings, detailed description and examples.

### BRIEF DESCRIPTION OF THE DRAWINGS

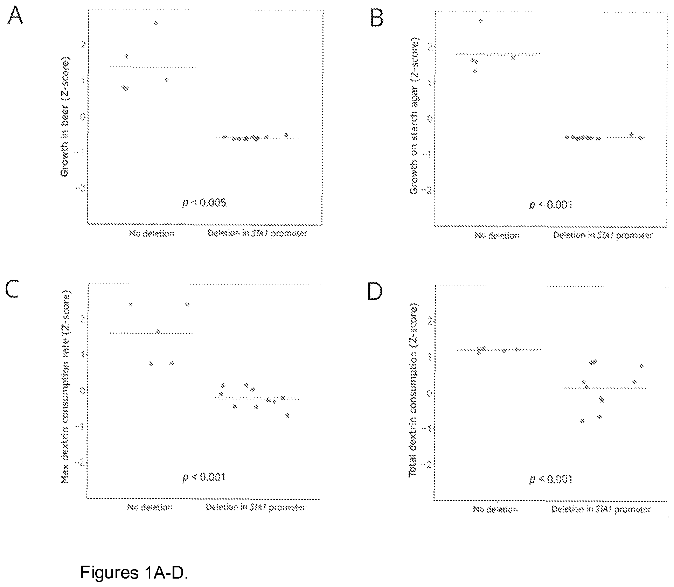

Figures 1A-D show the results of screening the diastatic ability. Diastatic assays show that despite carrying the *STA1* gene, only five out of 15 strains were able to grow in beer and on starch agar (Figure 1). The strains with the deletion in the promoter of STA1 gene exhibited significantly less diastatic ability in all tests (Figure 1A: growth in beer; Figure 1B: growth on starch agar; Figures 1C-D: fermentation of dextrin) between STA1+ strains with no deletion in the STA1 promoter compared to strains with a deletion in the STA1 promoter. The statistical significance between the two groups was tested with the Mann-Whitney U test.

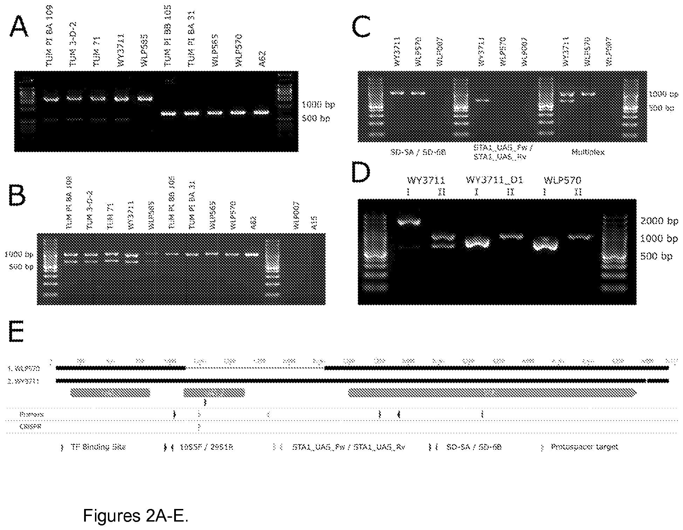

Figures 2A-E reveal a specific deletion in the promoter of *STA1* gene. A 1162 bp deletion was shown upstream of *STA1* (-1370 to -209 from the start codon), i.e. in the promoter of *STA1* gene, in 10 out of the 15 yeast strains (Figure 2A). To improve the reliability of the molecular detection methods for diastatic S. *cerevisiae,* two new primer pairs were designed to bind within the 1162 bp region that is absent in the poorly diastatic *STA1*+ strains. These new primers can therefore be used to differentiate whether a strain that has tested positive for *STA1* with the SD-5A / SD-6B primers (Yamauchi et al. 1998) is likely to be highly diastatic or not. The first primer pair (STA1_UAS_Fw / STA1_UAS_Rv) was designed to produce a 599 bp amplicon, and can be used together with the SD-5A / SD-6B primers in a single multiplex PCR reaction (Figures 2B-C). To confirm that the 1162 bp deletion that was observed in the *STA1* promoter had a negative effect on diastatic ability and *STA1* expression, we performed reverse engineering aided by the CRISPR/Cas9 system. The gRNA protospacer sequence was designed to cause a double-stranded break within the region to be deleted (Figure 2E). The 1162 bp region (-1370 to -209 upstream of the *STA1* open reading frame) was deleted from the highly diastatic WY3711 strain by transformation with a Cas9- and gRNA-expressing plasmid and an 80 bp double-stranded repair oligo. PCR using primer pairs binding within (STA1_UAS_Fw / STA1_UAS_Rv) and outside the deleted region (1055F / 2951R) was used to confirm successful deletion (Figure 2D).

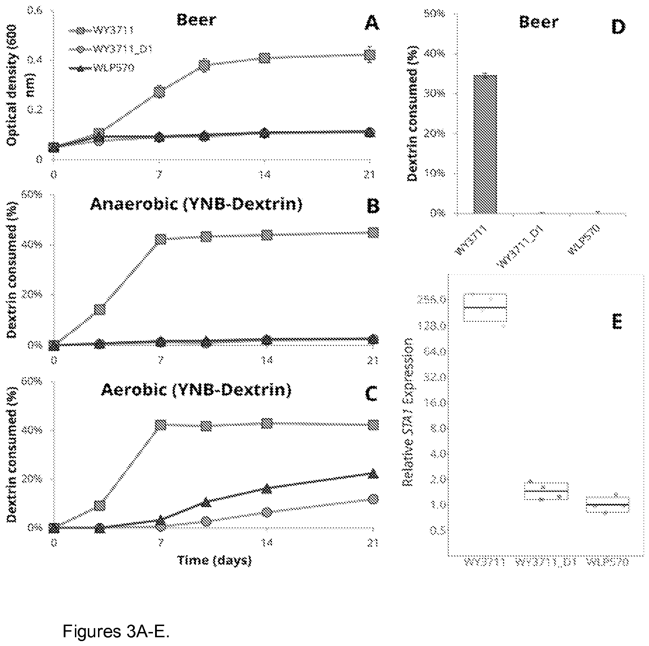

Figures 3A-E show phenotypic results of the reverse engineered strain, WY3711_D1. Three STA1+ strains were compared: WY3711 (no deletion in STA1 promoter), WY3711_D1 (CRISPR-mediated deletion in STA1 promoter), and WLP570 (natural deletion in STA1 promoter). (Figure 3A) The optical density (600 nm) when strains were inoculated into beer at a starting value of 0.1. Values for WY3711 were different from the two other strains starting from day 7 (p < 0.01 by two-tailed Student's t-test). (Figures 3B-C) The amount of dextrin consumed from YNB-Dextrin media in anaerobic (3B) and aerobic (3C) conditions. Values for WY3711 were different from the two other strains starting from day 4 (p < 0.01 by two-tailed Student's t-test). (Figure 3D) The amount of dextrin consumed from the beer (3A) after 3 weeks of incubation. (Figure 3E) The relative expression of STA1 (normalized to ALG9 and UBC6) determined by RT-qPCR in derepressed conditions. Points indicate values from four biological replicates and boxes indicate the mean and standard deviation. Values for WY3711 were significantly higher than those of the two other strains (p < 0.01 by two-tailed Student's t-test).

### SEQUENCE LISTING

| | |
|---|---|
| SEQ ID NO:1: | nucleotide sequence of *STA1* (*S. cerevisiae*) promoter and open reading frame, wherein a deletion may comprise or a deletion may be within nucleotides 1127-2288 |
| SEQ ID NO: 2 | oligonucleotide, primer used for the detection of intact *STA1* promoter, STA1_UAS_Fw |
| SEQ ID NO: 3 | oligonucleotide, primer used for the detection of intact *STA1* promoter, STA1_UAS_Rv |
| SEQ ID NO: 4 | oligonucleotide, primer used for the detection of intact *STA1* promoter, STA1_UAS_Q_Fw |
| SEQ ID NO: 5 | oligonucleotide, primer used for the detection of *STA1,* SD-5A |
| SEQ ID NO: 6 | oligonucleotide, primer used for the detection of *STA1,* SD-6B |
| SEQ ID NO: 7 | oligonucleotide, primer used for amplification and sequencing of *STA1,* STA1_Full_Fw |
| SEQ ID NO: 8 | oligonucleotide, primer used for amplification and sequencing of *STA1,* STA1_Full_Rv |
| SEQ ID NO: 9 | oligonucleotide, primer used for amplification and sequencing of *STA1,* STA1_24_F |
| SEQ ID NO: 10 | oligonucleotide, primer used for amplification and sequencing of *STA1,* STA1_546_R |
| SEQ ID NO: 11 | oligonucleotide, primer used for amplification and sequencing of *STA1,* STA1_482_F |
| SEQ ID NO: 12 | oligonucleotide, primer used for amplification and sequencing of *STA1,* STA1_1148_R |
| SEQ ID NO: 13 | oligonucleotide, primer used for amplification and sequencing of *STA1,* STA1_1055_F |
| SEQ ID NO: 14 | oligonucleotide, primer used for amplification and sequencing of *STA1,* STA1_1768_R |
| SEQ ID NO: 15 | oligonucleotide, primer used for amplification and sequencing of *STA1,* STA1_1671_F |
| SEQ ID NO: 16 | oligonucleotide, primer used for amplification and sequencing of *STA1*, STA1_2384_R |
| SEQ ID NO: 17 | oligonucleotide, primer used for amplification and sequencing of *STA1*, STA1_2267_F |
| SEQ ID NO: 18 | oligonucleotide, primer used for amplification and sequencing of *STA1*, STA1_2951_R |
| SEQ ID NO: 19 | oligonucleotide, primer used for amplification and sequencing of *STA1*, STA1_2901_F |
| SEQ ID NO: 20 | oligonucleotide, primer used for amplification and sequencing of *STA1,* STA1_3550_R |
| SEQ ID NO: 21 | oligonucleotide, primer used for amplification and sequencing of *STA1*, STA1_3474_F |
| SEQ ID NO: 22 | oligonucleotide, primer used for amplification and sequencing of *STA1,* STA1_4169_R |
| SEQ ID NO: 23 | oligonucleotide, primer used for amplification and sequencing of *STA1*, STA1_4051_F |
| SEQ ID NO: 24 | oligonucleotide, primer used for amplification and sequencing of *STA1,* STA1_4746_R |
| SEQ ID NO: 25 | oligonucleotide, primer used for amplification and sequencing of *STA1,* STA1_4657_F |
| SEQ ID NO: 26 | oligonucleotide, primer used for amplification and sequencing of *STA1,* STA1_5201_R |
| SEQ ID NO: 27 | CRISPR/Cas9 repair oligonucleotide, repair oligo_promoter deletion |

### DETAILED DESCRIPTION OF THE INVENTION

The present invention relates to a method for determining a yeast having extracellular glucoamylase STA1p activity in a sample, or more specifically to a method for genetically differentiating diastatic and non-diastatic yeasts carrying the *STA1* gene. In one embodiment said method comprises detecting the presence or absence of an alteration, more specifically a deletion, in the promoter of *STA1* gene in a sample.

In one embodiment the presence of the deletion in the promoter of *STA1* gene indicates decreased diastatic ability of the yeast compared to a yeast having *STA1* gene without a deletion in the promoter region. In one embodiment the presence of the deletion in the promoter of *STA1* gene indicates decreased extracellular glucoamylase STA1p activity of the yeast, e.g. when compared to a yeast (such as a corresponding yeast) having *STA1* gene without a deletion in the promoter region. Indeed, the inventors of the present disclosure were able to show that said deletion in the promoter of *STA1* gene greatly decreases diastatic ability and the expression of *STA1.*

In one embodiment the absence of the deletion in the promoter of *STA1* gene indicates diastatic ability, e.g. high diastatic ability, of the yeast. In one embodiment the absence of the deletion in the promoter of *STA1* gene indicates the presence of extracellular glucoamylase STA1p activity of the yeast.

Indeed, the present invention enables determining the presence of a yeast having e.g. high, low or intermediate extracellular glucoamylase STA1p activity. In a specific embodiment yeast strains with the deletion have low or very low levels of STA1p activity but it is not completely absent.

As used herein *STA1* gene refers to a gene encoding STA1p, which is a glucoamylase (glucan 1,4-alpha-glucosidase), i.e. an exoamylase which cleaves 1,4-α-glycosidic bonds from the nonreducing end of the glycosidic chains releasing D-glucose, thus increasing the content of fermentable carbohydrates and reducing the nonfermentable dextrins. Glucoamylase is classified as EC 3.2.1.3. In one embodiment all isozymes, isoforms and variants are included with the scope of STA1p. As used herein "glucoamylase STA1p activity" refers to exoamylase activity cleaving 1,4-α-glycosidic bonds from the nonreducing end of the glycosidic chains releasing D-glucose. By the inventors of the present disclosure it was proved that STA1p appears to have a central role in enabling maltotriose use during wort fermentation.

As an example, STA1p protein of the *S*. *cerevisiae* is identified in the scientific article of Kleinman et al. (1988, Biochem J, 249: 163-170). STA1p refers to not only *S*. *cerevisiae* STA1p but also to any other homologue from any yeast. *S*. *cerevisiae STA1* gene is described for example in the article of Yamashita et al. (1985, J Bacteriol, 161: 567-573). Examples of suitable open reading frames (ORF) or gene accession numbers include but are not limited to ORF of *S*. *cerevisiae* and/or gene accession number Genbank X02649.1.

As used herein "decreased activity" or "low activity" of glucoamylase STA1p refers to the presence of decreased activity, less activity, if any, or low activity of said protein STA1p, or decreased or low total protein activity of a yeast or cell, e.g. when compared to a yeast or cell without a deletion in the promoter region of *STA1* gene. "Decreased activity or low activity" also comprises a situation, wherein a protein is totally inactivated and e.g. a yeast or cell has no activity of said protein. "Decreased or low protein activity" may result e.g. from down-regulated polypeptide expression, down-regulated gene expression, decreased level of functional glucoamylases, lack of at least part of the gene, lack of protein, the presence of deletions or attenuating mutations in a gene encoding said glucoamylase STA1p and/or the presence of mutations (e.g. insertions, deletions, substitutions) in STA1p. As used herein "increased activity" or "high activity" of glucoamylase STA1p refers to the presence of higher activity, more activity or high activity of a protein, or higher or high total protein activity of a yeast or cell, e.g. when compared to a yeast or cell with a deletion in the promoter region of *STA1* gene. "Increased or high protein activity" may result e.g. from up-regulated or normal polypeptide expression, up-regulated or normal gene expression, increased or normal level of functional glucoamylases, lack of deletions or attenuating mutations in the gene encoding said glucoamylase STA1p, and/or the presence of enhancing mutations in STA1p or *STA1* gene.

In a specific embodiment "decreased activity of glucoamylases" or "low activity of glucoamylases" or "decreased level of functional glucoamylases" refers to a situation, wherein a yeast or cell comprising or lacking glucoamylases is not able to grow in beer, e.g. during 3 weeks incubation at 25°C (Figure 3A, 3D). In another specific embodiment "increased activity of glucoamylases" or "high or normal activity of glucoamylases" or "increased or normal level of functional glucoamylases" refers to a situation, wherein a yeast or cell comprising glucoamylases has the ability to grow in beer, and consume dextrin from the beer, e.g. during 3 weeks incubation at 25°C. (Figure 3A, 3D).

In one embodiment of the present invention the yeast comprises a deletion in the promoter area of *STA1* gene, thereby reducing expression of said gene. In a very specific embodiment the deleted region comprises an upstream activation sequence, shown to increase expression of the *STA1* gene.

As used herein "extracellular activity" refers to an activity, which is present outside of the cell. Said expression is opposite to the expression intracellular.

In a specific embodiment of the invention the presence or absence of the deletion in the promoter area of *STA1* gene is detected by measuring the level of STA1p activity in a sample. Optionally a molecular method or a microbiological method can be used in combination with measuring the level of STA1p activity in a sample.

Glucoamylase STA1p activity can be measured by any method known in the field. The presence, absence or level of protein activities e.g. in a cell or yeast can be detected or measured by any suitable method known in the art. Non-limiting examples of suitable detection and/or measuring methods include enzymatic assays, PCR based assays (e.g., multiplex PCR, qPCR, RT-PCR, RT-qPCR), immunological detection methods (e.g., antibodies specific for said proteins), or any method described in the examples of the present disclosure or related to any of figures 1-3 and 6, and any combination thereof. Methods of the examples include but are not limited to PCR based assays (e.g., multiplex PCR, qPCR, RT-PCR, RT-qPCR), HPLC, differential refractometer, organic acid analysis columns, sequencing, visualization e.g. on agarose gels, dextrin fermentation or consumption and analyzing, growth on starch agar and analyzing, growth in beer (spoilage potential) and analyzing, wort fermentation and analyzing, analyzing sugar consumption optionally together with analyzing dextrin consumption, and any combination thereof.

Expression or increased expression can be proved for example by western, northern or southern blotting or quantitative PCR or any other suitable method known to a person skilled in the art.

In one embodiment of the invention the presence of the deletion in the promoter of *STA1* gene results in decreased ethanol formation, carbon dioxide formation, hydrolysis of malto-oligomers and/or diastatic capability of the yeast compared to another yeast lacking the deletion in the promoter of *STA1* gene.

The method of the present invention, wherein the method comprises detecting the presence or absence of a deletion in the promoter of *STA1* gene in a sample, may further comprise detection of one or more genes or any alterations thereof. In one embodiment the method further comprises detecting the presence or absence of *STA1* gene. E.g. if two different probes or primer pairs are utilized, it is possible to detect strains, which carry the *STA1* gene with the promoter deletion, and those strains, which carry the *STA1* gene. E.g. by using STA1_UAS_Fw (SEQ ID NO: 2) and/or STA1_UAS_Rv (SEQ ID NO: 3) primers either alone or together with SD-5A (SEQ ID NO: 5) and/or SD-6B primers (SEQ ID NO: 6) (see table 2), it is possible to determine the presence or absence of a deletion in the promoter area of *STA1* gene. Alternatively e.g. STA1_UAS_Q_Fw (SEQ ID NO: 4) and/or STA1_UAS_Rv (SEQ ID NO: 3) primers either alone or together with other primers can be used to determine the presence or absence of a deletion in the promoter area of *STA1* gene.

Genetic methods for determining a yeast having extracellular glucoamylase STA1p activity in a sample may also be combined with any other methods known in the field of biotechnology. E.g. in a specific embodiment the method of the present invention may optionally further comprise a microbiological detection method such as detection through culturing yeasts on specialized selective growth media.

In a very specific embodiment the non-spoilage yeast strains which carry the STA1 gene (i.e. those that get flagged as false positives using the currently available genetic test) have a 300-1600 bp deletion (such as 500-1400 bp, 800-1300 bp, or 1000-1200 bp deletion, e.g. 1162 bp deletion) in the promoter region of the STA1 gene. This region contains an upstream activation sequence, which has been shown to increase expression of the STA1 gene. In a specific embodiment the deletion comprises or is a deletion of nucleotides 1127-2288 as numbered in SEQ ID NO: 1, or the deletion is a deletion within nucleotides 1127-2288 as numbered in SEQ ID NO: 1.

Any method or tools known in the field of biotechnology can be utilized for detecting the deletion of the *STA1* gene, e.g. a deletion in the promoter area of said gene. In one embodiment the presence or absence of the deletion is detected by a molecular method, optionally by utilizing one or more primers and/or a probe. In one embodiment the molecular method is a polymerase chain reaction (PCR, e.g. multiplex PCR, qPCR, RT-PCR, RT-qPCR) and the method optionally further comprises agarose gel electrophoresis of the product obtained from the PCR. In a very specific method the PCR is a quantitative PCR. In another embodiment the molecular method is a Southern blot, northern blot or whole genome sequencing. In a very specific embodiment the presence or absence of the deletion is detected by a molecular method in combination with a microbiological method or with analysing the level of STA1p activity in a sample.

In one embodiment the method of the present invention comprises allowing a primer or probe capable of hybridizing with the promoter of *STA1* gene to contact or hybridize with the polynucleotide, DNA or RNA of the sample or obtained from the sample, e.g. in stringent hybridization conditions. In a specific embodiment of the invention detecting the presence or absence of the deletion in the promoter of *STA1* gene is carried out by allowing a primer or probe capable of hybridizing with the promoter of *STA1* gene within nucleotides 1127-2288 as numbered in SEQ ID NO: 1 to hybridize with the DNA or RNA obtained from the sample. In a very specific embodiment a further primer and/or probe are used for detecting STA1 and/or another gene.

The present invention further relates to a primer, primer pair or probe that binds specifically to the *STA1* gene. Said primer or probe can be used to differentiate spoilage and non-spoilage yeast strains, said non-spoilage strains comprising a deleted promoter region, optionally e.g. highly and poorly diastatic strains. A primer of the present invention is capable of hybridizing with *STA1* gene of a yeast, more specifically with the promoter of *STA1* gene, e.g. within nucleotides 1127-2288 as numbered in SEQ ID NO: 1. A probe of the present invention is capable of hybridizing with *STA1* gene of a yeast, more specifically with the promoter of *STA1* gene, e.g. within or at least partially within nucleotides 1127-2288 as numbered in SEQ ID NO: 1. In other words, in one embodiment a primer or probe, which is not able to bind *STA1* in the presence of the promoter deletion, is suitable for the present invention. "At least partially" refers to a situation wherein some nucleotides of a probe hybridize with *STA1* gene within the nucleotides 1127-2288 as numbered in SEQ ID NO: 1, and some nucleotides hybridize with *STA1* gene outside of the region of nucleotides 1127-2288 as numbered in SEQ ID NO: 1, in a situation wherein there is no deletion in the promoter area of *STA1.*

A primer pair of the present invention comprises two primers capable of hybridizing with *STA1* gene of a yeast, wherein at least one of said primers is capable of hybridizing with the promoter of *STA1* gene, e.g. within nucleotides 1127-2288 as numbered in SEQ ID NO: 1. In one embodiment both of two primers are capable of hybridizing with the promoter of *STA1* gene, e.g. within nucleotides 1127-2288 as numbered in SEQ ID NO: 1. In another embodiment the other primer of the primer pair hybridizes with the *STA1* gene outside the promoter region nucleotides 1127-2288 as numbered in SEQ ID NO: 1.

Optionally the primer pair of the present invention can be used together with other primers or probes, e.g. a primer pair for detecting STA1 in a PCR reaction. The aim may thus be to detect the presence of both the STA1 gene and the upstream activation sequence in the promoter which renders the strain a spoilage strain.

In a specific embodiment of the method or for the primer pairs of the invention the PCR reaction for a spoilage strain carrying *STA1* may e.g. result in two PCR products of different sizes, which optionally can be separated on an agarose gel using electrophoresis, and/or the PCR reaction for non-spoilage strains carrying *STA1* may result in only a single PCR product. In another embodiment the PCR reaction for a spoilage strain carrying *STA1* may e.g. result in one PCR product, which optionally can be shown on an agarose gel using electrophoresis, and/or the PCR reaction for non-spoilage strains carrying *STA1* may result in no PCR products. As an example, STA1_UAS_Fw (SEQ ID NO: 2) and/or STA1_UAS_Rv (SEQ ID NO: 3) primers (see table 2) can be used to obtain one PCR product in a case, wherein there is no deletion in the promoter of *STA1* gene (e.g. visualized as one band in the agarose gel) and no PCR products in a case, wherein there are deletions in the promoter of *STA1* gene. In another specific example, STA1_UAS_Fw (SEQ ID NO: 2) and STA1_UAS_Rv (SEQ ID NO: 3) primers can be used together with a further primer or primers such as SD-5A (SEQ ID NO: 5) and/or SD-6B (SEQ ID NO: 6) (see table 2). In such a case, two PCR products can be obtained in a case, wherein there is no deletion in the promoter of *STA1* gene (e.g. visualized as two bands in the agarose gel) and one PCR product in a case, wherein there is a deletion in the promoter of *STA1* gene.

In the method or tools of the present invention a primer, primer pair or probe capable of hybridizing *STA1* gene of the yeast can be of any length, optionally capable of hybridizing in stringent conditions. In one embodiment of the method, primer, primer pair, probe or kit of the invention the primer or probe capable of hybridizing *STA1* gene of the yeast is at least 15 nucleotides, e.g. 18-50 nucleotides, 18-40 nucleotides, 18-30 nucleotides, 18-25 nucleotides, 19-24 nucleotides, 20-23 nucleotides, or 20-22 nucleotides. In a specific embodiment of the method, primer, primer pair or kit, one or several primers capable of hybridizing with the promoter of *STA1* gene are selected from the group consisting of a nucleotide sequence as shown in SEQ ID NO: 2, a nucleotide sequence as shown in SEQ ID NO: 3, a nucleotide sequence as shown in SEQ ID NO: 4, and any combination thereof.

A person skilled in the art is able to design suitable hybridizing conditions for the primers or probes. In one embodiment hybridization of the present methods, primers or probes is performed under stringent conditions that allow specific binding between a primer or probe and the target nucleotide sequence of *STA1* gene. Such stringent conditions for hybridization are sequence-dependent and varied depending on environmental parameters. In a specific embodiment the stringent conditions are high stringent conditions. For example, stringent hybridization conditions can comprise 50 - 80°C (such as 55 - 75°C or 60 - 72°C) at least 10-30 sec (e.g. at least 15 sec), or at least one or more minutes, e.g. one or more times as is the case during a PCR reaction.

A kit of the present invention for genetic testing comprises a primer, primer pair or probe of the invention and optionally reagents for performing said genetic testing. As used herein "genetic testing" refers to testing the presence or absence of a deletion in the promoter of *STA1* gene. In a specific embodiment the kit is for the method of the present invention.

Reagents suitable to be used for performing genetic testing include but are not limited to reaction solutions (e.g. solutions for a PCT based reaction) and/or washing solutions, buffers and enzymes. In one embodiment additional tools for detecting the presence or absence of a deletion in the promoter of *STA1* gene include but are not limited to detection means selected from the group comprising labels, colouring agents, and antibodies or antigen binding fragments specific for STA1p. Detection mode of the method or kit of the present invention can be any conventional detection mode including but not limited to e.g. colorimetric or fluorescent detection modes.

In general, the presence or absence of a deletion in the promoter of *STA1* gene can be detected by any suitable method or tools known in the art suitable for detecting genetic alterations. Optionally the kit or method of the present invention may also comprise use of any suitable statistical methods or tools or instructions related thereto, respectively, known to a person skilled in the art.

In one embodiment the kit further comprises instructions for carrying out said genetic testing. Said instructions may include but are not limited to instructions selected from the group consisting of instructions for carrying out the genetic testing, instructions for carrying out a PCR based method, conditions of the PCR reaction, instructions for separating the PCR products e.g. in electrophoresis, and instructions for interpreting the results (e.g. results of the PCR based reaction optionally wherein the PCR products have been separated by electrophoresis and are visualized on an agarose gel).

In a very specific embodiment of the invention the kit comprises a primer, primer pair or probe of the invention and optionally reagents for performing said genetic testing, and optionally control samples and optionally instructions for carrying out genetic testing. Control samples may be selected e.g. from a sample comprising a deletion in the promoter region of *STA1* gene, a sample lacking a deletion in the promoter region of *STA1* gene, or combination thereof. In one embodiment the kit of the present invention comprises tools for detecting *STA1* gene and/or other genes or alterations thereof.

In one embodiment of the invention the primer, primer pair, probe or kit is for detecting the presence or absence of a deletion in the promoter of *STA1* gene in a yeast, optionally in a sample.

A sample which can be analyzed with the method or tools of the present invention includes any sample preferably comprising genetic material or a yeast. In one embodiment the sample is a food sample, biological sample, environmental sample, sample from a facility or surface, air sample or water sample, or selected from the group consisting of a product comprising a substrate for glucoamylase activity, a product of brewing industry, alcoholic beverage, beer, rice wine, sake, and soy sauce. As used herein "a substrate for glucoamylase activity" refers to any substrate comprising glycosidic chains having 1,4-α-glycosidic bonds in the nonreducing end. Said substrate can be selected e.g. from the group consisting of starch, maltodextrin and oligosaccharides.

In one embodiment the sample has been obtained from food (including drinks and beverages), which is to be packed in a bottle, can, keg, box or package. Indeed, the presence of e.g. normal, high or significant extracellular glucoamylase STA1p activity in said sample causes high or increased carbon dioxide and ethanol levels thereby increasing the risk of exploding bottles, cans, keg, box or packages.

The methods and tools of the present invention are for yeasts. Therefore, *STA1* genes utilized in the present invention are yeast genes. In one embodiment of the invention the yeast belongs to a genus *Saccharomyces,* e.g. the yeast is selected from the group consisting of species known as, or formerly known as, S. *arboricolus, S. bayanus, S. boulardii, S. bulderi, S. cariocanus, S. cariocus, S. cerevisiae, S. chevalieri, S. dairenensis, S. ellipsoideus, S. eubayanus, S. exiguus, S. florentinus, S. fragilis, S. kluyveri, S. kudriavzevii, S. jurei, S. martiniae, S. mikatae, S. monacensis, S. norbensis, S. paradoxus, S. pastorianus, S. spencerorum, S. turicensis, S. unisporus, S. uvarum,* and S. *zonatus.* In a specific embodiment said yeast is S. *cerevisiae.*

Also, the present invention relates to use of the primer, primer pair, probe or kit of the present invention for detecting the presence or absence of a deletion in the promoter of *STA1* gene in a yeast.

And still furthermore, the present invention concerns a genetically modified yeast that has been genetically modified to decrease extracellular glucoamylase STA1p activity. Also, the present invention concerns a yeast that has been modified by adaptive evolution strategy to decrease extracellular glucoamylase STA1p activity. The present invention relates to a (genetically) modified yeast comprising a deletion in the promoter of *STA1* gene, e.g. said deletion comprising or being within nucleotides 1127-2288 as numbered in SEQ ID NO: 1. Methods for making any genetic modifications or modifying microorganisms by adaptive evolution strategy are generally well known by a person skilled in the art and are described in various practical manuals describing laboratory molecular techniques. The knowledge gained during the study of the present disclosure can be used for obtaining modified yeasts comprising specific alterations. The construction of a yeast in which one or more genes or parts thereof are genetically modified is within the skills of an artisan. Some examples of the general procedure and specific embodiments are described in the Examples chapter. In one embodiment of the invention the genetic modification has been carried out by a CRISPR/Cas9 based method.

In a specific embodiment the yeast that has been genetically modified to decrease extracellular glucoamylase STA1p activity in a yeast belongs to a genus *Saccharomyces* or in a more specific embodiment said yeast is S. *cerevisiae.*

The yeast of the present invention may also contain other genetic modifications than the modifications of *STA1* gene, e.g. deletions in the promoter region of the *STA1* gene as specifically described in this disclosure.

As used in the present disclosure, the terms "polypeptide" and "protein" are used interchangeably to refer to polymers of amino acids of any length.

As used herein "polynucleotide" refers to any polynucleotide, such as single or double-stranded DNA (genomic DNA or cDNA) or RNA, comprising a nucleic acid sequence encoding a polypeptide in question or a conservative sequence variant thereof. Conservative nucleotide sequence variants (i.e. nucleotide sequence modifications, which do not significantly alter biological properties of the encoded polypeptide) include variants arising from the degeneration of the genetic code and from silent mutations.

It will be obvious to a person skilled in the art that, as the technology advances, the inventive concept can be implemented in various ways. The invention and its embodiments are not limited to the examples described below but may vary within the scope of the claims.

### EXAMPLES

### Materials & Methods

### Yeast strains

A list of strains used in this study can be found in Table 1.

**Table 1 - Yeast strains used in the study.**

| **Code** | **Alternative name** | **Species** | ***STA1*** | **1162 bp deletion in *STA1* promoter** | **Source** |
|---|---|---|---|---|---|
| A15 | VTT-A83015 | *S*. *pastorianus* | - | NA | VTT Culture Collection |
| A60 | VTT-A75060 | *S*. *cerevisiae* | - | NA | VTT Culture Collection |
| WLP007 | | *S*. *cerevisiae* | - | NA | White Labs Inc. |
| WLP023 | | *S*. *cerevisiae* | - | NA | White Labs Inc. |
| TUM PI BA 109 | | *S*. *cerevisiae* | + | - | BLQ Weihenstephan |
| TUM 3-D-2 | | *S*. *cerevisiae* | + | - | BLQ Weihenstephan |
| TUM PI BA 45 | | *S*. *cerevisiae* | + | + | BLQ Weihenstephan |
| TUM PI BB 105 | | *S*. *cerevisiae* | + | + | BLQ Weihenstephan |
| TUM PI BA 31 | | *S*. *cerevisiae* | + | + | BLQ Weihenstephan |
| TUM 71 | | *S*. *cerevisiae* | + | - | BLQ Weihenstephan |
| TUM 3-H-2 | | *S*. *cerevisiae* | + | - | BLQ Weihenstephan |
| WY3711 | | *S*. *cerevisiae* | + | - | Wyeast Laboratories |
| WLP565 | | *S*. *cerevisiae* | + | + | White Labs Inc. |
| WLP566 | | S. *cerevisiae* | + | + | White Labs Inc. |
| WLP570 | | S. *cerevisiae* | + | + | White Labs Inc. |
| WLP644 | | S. *cerevisiae* | + | + | White Labs Inc. |
| Ontario Farmhouse | | S. *cerevisiae* | + | + | Escarpment Laboratories |
| Wild Thing | | *S*. *cerevisiae* | + | + | Escarpment Laboratories |
| A62 | VTT-A81062 | *S*. *cerevisiae* | + | + | VTT Culture Collection |

### Dextrin fermentation

The ability to ferment dextrin was tested in minimal growth media with dextrin as the sole carbon source. Strains were grown overnight in YP-Glucose (2%), after which 20 µL of overnight culture was used to inoculate 2 mL microcentrifuge tubes containing 1 mL of dextrin media (0.67% YNB without amino acids, 1% dextrin from potato starch). The tubes were incubated at room temperature for 3 weeks. Samples were drawn each week and analysed with HPLC to determine the amount of dextrin consumed.

### Growth on starch agar

The ability to grow on solid media containing starch as the sole carbon source was tested on agar plates (Meier-Dörnberg et al., 2018). Agar plates were prepared containing 0.67% YNB /wo amino acids, 1.5% soluble starch and 40 mg/L bromophenol blue. pH was adjusted to 5.2 with 0.1M HCl. Yeast strains were grown overnight in YP-Glucose, washed twice with sterile MQ-H₂O, and then resuspended to an OD600 of 1. Aliquots of yeast suspension (100 µL) were spread out on agar plates. The plates were incubated in an anaerobic jar (Anoxomat AN2CTS, Mart Microbiology) at 25 °C for four weeks.

### Growth in beer

The spoilage potential of *STA1*+ strains was assessed by growing them in beer. Beer was produced by inoculating one litre of 10 °Plato all-malt wort with *S*. *pastorianus* A15 and fermenting at 25 °C for one week. The beer was centrifuged and sterile-filtered (0.45 µm, followed by 0.22 µm). The beer was analysed with HPLC and determined to contain less than 1 g/L each of maltose and maltotriose. Yeast strains were grown overnight in YP-Glucose, washed twice with sterile MQ-H₂O, and then resuspended to an OD600 of 1. Sterile-filtered beer (4750 µL) was inoculated with 250 µL of pre-culture for a starting OD600 of 0.05. The beer cultivations were incubated statically in an anaerobic jar (Anoxomat AN2CTS, Mart Microbiology) at 25 °C for three weeks. The optical density was monitored once a week, and the final amount of dextrin consumed was analysed with HPLC after three weeks.

### Wort fermentation and analysis

Concentrations of fermentable sugars and dextrin were measured by HPLC using a Waters 2695 Separation Module and Waters System Interphase Module liquid chromatograph coupled with a Waters 2414 differential refractometer (Waters Co., Milford, MA, USA). An Aminex HPX-87H organic acid analysis column (300 × 7.8 mm, Bio-Rad) was equilibrated with 5 mM H₂SO₄ (Titrisol, Merck, Germany) in water at 55 °C and samples were eluted with 5 mM H₂SO₄ in water at a 0.3 ml/min flow rate.

### Sequencing of STA1

The *STA1* open reading frame and promoter (2.5 kb upstream) were amplified by PCR and then sequenced using Sanger sequencing. A nested PCR approach was used to prevent PCR primers from amplifying fragments from *FLO11* and *SGA1.* First, a 5.2 kb amplicon was produced using primers STA1_Full_Fw and STA1_Full_Rv (Table 2). The amplicon was diluted 1:500 in MQ-H₂O and used as template DNA for the ten PCR reactions described in Table 2. All PCR reactions were carried out with Phusion High-Fidelity PCR Master Mix with HF Buffer (Thermo Scientific) and primer concentrations of 0.5 µM. Amplicons were cleaned using the QIAquick PCR purification kit of Qiagen (Hilden, Germany), and sequenced at Seqlab-Microsynth (Goettingen, Germany). Sequences were aligned in Geneious 10.0.9 (Biomatters).

**Table 2 - Oligonucleotides used in the study.**

| **Name** | **Sequence (5' > 3')** | **Reference** |
|---|---|---|
| **Primers used for amplification and sequencing of *STA1* ORF and upstream region** | | |
| STA1_Full_Fw | TGGAATGAACAGCGCCAAGT | This study (SEQ ID NO: 7) |
| STA1_Full_Rv | AGTGGGAGAAAAAGGTGGCC | This study (SEQ ID NO: 8) |
| STA1_24_F | AATGAACAGCGCCAAGTAGC | This study (SEQ ID NO: 9) |
| STA1_546_R | TTGAAAGCTATGTGCAGTTGG | This study (SEQ ID NO: 10) |
| STA1_482_F | TGTCCCCTAATGTATCCCTCA | This study (SEQ ID NO: 11) |
| STA1_1148_R | AAATCTTACCCGTGGATCCTTT | This study (SEQ ID NO: 12) |
| STA1_1055_F | CCCAAAATTCATTCGTAGCC | This study (SEQ ID NO: 13) |
| STA1_1768_R | TATGCGATGTCCCAGTACGA | This study (SEQ ID NO: 14) |
| STA1_1671_F | TGTCAGGCATTGCACAAACT | This study (SEQ ID NO: 15) |
| STA1_2384_R | CAATTGAGAACCCTTCAACAA | This study (SEQ ID NO: 16) |
| STA1_2267_F | AGGGCAGTTTTATTTACCTTAACA | This study (SEQ ID NO: 17) |
| STA1_2951_R | AAGTGGTTGTTGATTCCGATG | This study (SEQ ID NO: 18) |
| STA1_2901_F | TCCATGTTCAACCAGTCCAA | This study (SEQ ID NO: 19) |
| STA1_3550_R | CTGTCGCTGGAGCCACTC | This study (SEQ ID NO: 20) |
| STA1_3474_F | CTTGATGAATGGGACAGTGG | This study (SEQ ID NO: 21) |
| STA1_4169_R | GACCGTTCTGAGGCGTTAAA | This study (SEQ ID NO: 22) |
| STA1_4051_F | TGGAATTCTTCCGGATTTGA | This study (SEQ ID NO: 23) |
| STA1_4746_R | GGTTTGATTGAAGGCAGGTG | This study (SEQ ID NO: 24) |
| STA1_4657_F | AGCGAGCTGGTATTCTCCAA | This study (SEQ ID NO: 25) |
| STA1_5201_R | ACACGCTTTGGACATCATCA | This study (SEQ ID NO: 26) |

| **Primers used for the detection of *STA1*** | | |
|---|---|---|
| SD-5A | CAACTACGACTTCTGTCATA | Yamauchi et al., 1998, (SEQ ID NO: 5) |
| SD-6B | GATGGTGACGCAATCACGA | Yamauchi et al., 1998, (SEQ ID NO: 6) |

| **Primers used for the detection of intact *STA1* promoter** | | |
|---|---|---|
| STA1_UAS_Fw | CCTGGCTCAAATTAAACTTTCG | This study, (SEQ ID NO: 2) |
| STA1_UAS_Rv | ACCACCAATAGGCAATAGAAA | This study, (SEQ ID NO: 3) |
| STA1_UAS_Q_Fw | CAAGGCAATCAGTTAAAAGA | This study, (SEQ ID NO: 4) |

| **Primers used in quantitative PCR** | | |
|---|---|---|
| Sdia-f | TTCCAACTGCACTAGTTCCTAGAGG | Brandl, 2006 |
| Sdia-r | GAGCTGAATGGAGTTGAAGATGG | Brandl, 2006 |
| UBC6 F | GATACTTGGAATCCTGGCTGGTCTGTCTC | Teste et al., 2009 |
| UBC6_R | AAAGGGTCTTCTGTTTCATCACCTGTATTT . GC | Teste et al., 2009 |
| ALG9 F | CACGGATAGTGGCTTTGGTGAACAATTAC | Teste et al., 2009 |
| ALG9_R | TATGATTATCTGGCAGCAGGAAAGAACTT GGG | Teste et al., 2009 |

| **CRISPR/Cas9 repair oligos** | | |
|---|---|---|
| repair_oligo_promoter_del etion | GACGGGGTATTATGAATAAAGGATCCACG GGTAAGATTTGCTGCGCTCTCTTCTAGTTC AAGAACGGATAACTCATAGAC | This study (SEQ ID NO: 27) |

### Multiplex PCR to detect presence of deletion in STA1 promoter

The presence of the *STA1* gene was tested with PCR using the previously published primers SD-5A and SD-6B (Yamauchi et al. 1998). These primers amplify an 868 bp fragment from the *STA1* gene. In addition, a new primer pair (STA1_UAS_Fw and STA1_UAS_Rv; Table 2) was designed to detect whether the *STA1*+ yeast strains had the full promoter sequence. Both primers bind within the 1162 bp region that was found to be deleted in the poorly diastatic strains, and they amplify a 599 bp fragment. Primers were tested in separate and multiplex PCR reactions. PCR reactions were carried out with Phusion High-Fidelity PCR Master Mix with HF Buffer (Thermo Scientific) and primer concentrations of 0.5 µM. The following PCR program was used: 98 °C 30 sec, (98 °C 10 sec, 63 °C 15 sec, 72 °C 30 sec) × 30 cycles, 72 °C 10 min. PCR products were separated and visualized on 1.0% agarose gels.

### Quantitative PCR to detect presence of deletion in STA1 promoter

The presence of the *STA1* gene was tested with quantitative PCR using the previously published primers Sdia-f and Sdia-r (Brandl, 2006). These primers amplify an 79 bp fragment from the *STA1* open reading frame. In addition, a new primer pair (STA1_UAS_Q_Fw and STA1_UAS_Rv; Table 2) was designed to detect whether the *STA1*+ yeast strains had the full promoter sequence. Both primers bind within the 1162 bp region that was found to be deleted in the poorly diastatic strains, and amplify a 223 bp fragment. The qPCR reactions were prepared with PerfeCTa SYBR^{®} Green SuperMix (QuantaBio, USA) and 0.3 µM of the primers (Table 2). The qPCR reactions were performed on a LightCycler^{®} 480 II instrument (Roche Diagnostics, Switzerland) in two technical replicates on 50 ng template DNA extracted with a YeaStar Genomic DNA kit (Zymo Research, USA). The following program was used: pre-incubation (95 °C for 3 min), amplification cycle repeated 45 times (95 °C for 15 s, 60 °C for 30 s, 72 °C for 20 s with a single fluorescence measurement), melting curve program (65-97 °C with continuous fluorescence measurement), and finally a cooling step to 40 °C.

### CRISPR/Cas9 mediated deletions

In order to confirm the effect of the 1162 bp deletion that was observed in the *STA1* promoter of the poorly diastatic strains, reverse engineering in the highly diastatic *S*. *cerevisiae* WY3711 strain was performed using the CRISPR/Cas9 system. Plasmid construction was carried out using the plasmid pCC-036 as backbone (Rantasalo et al., 2018, ACS Synth. Biol. 7, 6, 1573-1587). pCC-036 contains yeast codon-optimized Cas9 expressed under *TDH3p,* guiding RNA (gRNA) expressed under *SNR52p,* and *hygR* for selection on hygromycin. The gRNA protospacer sequence, TGGCTCAAATTAAACTTTCG, was designed using CCTop (Stemmer et al., 2015, PLoS One, 12(4), e0176619). The protospacer sequence was designed to induce a double-stranded break within the region to be deleted and was chosen for minimal off-target activity. A synthetic DNA fragment with the gRNA sequence was ordered from Integrated DNA Technologies, and introduced into the plasmid with restriction enzyme-based techniques (Thermo Scientific, Finland). The ligated plasmid was transformed into *E. coli* TOP10 by electroporation, and plasmid correctness was confirmed by Sanger sequencing. A 80 bp repair oligo (repair_oligo_promoter_deletion SEQ ID NO:27), consisting of adjacent 40 bp sequences homologous to those up- and downstream of the deleted region (Table 2), was also ordered from Integrated DNA Technologies.

Transformation of *S*. *cerevisiae* WY3711 was performed using a standard lithium acetate-based protocol with 40 minute incubation at 42 °C (Gietz and Woods, 2002, Methods Enzymol. Vol 350, Pages 87-96). Cells were transformed together with 3.6 µg of purified plasmid and 2.5 nmol of repair oligo. The transformed cells were selected on plates containing 300 mg/L Hygromycin B (Sigma-Aldrich). Colony PCR using primer pairs 1055F/2951R and STA1_UAS_Fw/STA1_UAS_Rv was used to confirm successful deletion in the transformed cells. Colonies from selection plates were replated three times onto YPD agar plates to encourage plasmid loss, after which they were stored at -80°C.

### STA1 transcript analysis by RT-qPCR

Strains were grown overnight in YP-Glucose, after which four replicate cultures per strain were started by inoculating 20 mL YPGE (1% yeast extract, 2% peptone, 3% glycerol and 2% ethanol) to a starting OD600 of 0.1. Cultures were overnight at 25 °C (OD600 varied between 2 and 4), after which RNA was isolated from pelleted yeast using hot formamide extraction (Shedlovskiy et al., 2017, RNA Biol. Vol 14, issue 12, pages 1722-1726). RNA was precipitated by first diluting 50 µL RNA solution with 25 µL 3M sodium acetate and 200 µL nuclease-free water, after which 825 µL ice-cold ethanol was added and solutions were stored overnight at - 20 °C. The solutions were centrifuged at 16000 × *g* for 30 minutes, after which the pellet was washed with ice-cold 75% ethanol and tubes were centrifuged again at 8000 × *g* for 5 minutes. The supernatant was removed and the pellet was allowed to air-dry for up to 30 minutes, after which the RNA pellet was dissolved in nuclease-free water. The RNA solution was treated with TURBO DNAse (DNA-Free kit, Invitrogen) and the DNAse was subsequently inactivated using the supplied inactivation reagent. RNA was quantified with a Qubit 2.0 fluorometer and its quality was assessed on 1.2% agarose gels (made in 1×TAE buffer).

250 ng of total RNA was reverse-transcribed using a qScript Flex cDNA kit (QuantaBio, USA), using a mixture of supplied oligo-dT and random primers according to kit instructions. The resulting cDNA was diluted 5-fold and 4 µL of diluted cDNA (corresponding to 10 ng of total RNA) was used as template in 20 µL qPCR reactions. The qPCR reactions were prepared with PerfeCTa SYBR^{®} Green SuperMix (QuantaBio, USA) and 0.3 µM of gene-specific primers (Table 2). In addition to using primers specific to *STA1,* reactions with primers for two house-keeping genes *(ALG9* and *UBC6*) were also performed (Teste et al., 2009, BMC Mol. Biol. 10:99). The qPCR reactions were performed on a LightCycler^{®} 480 II instrument (Roche Diagnostics, Switzerland) in two technical replicates on the reverse-transcribed RNA isolated from four biological replicates. The following program was used: pre-incubation (95 °C for 3 min), amplification cycle repeated 45 times (95 °C for 15 s, 60 °C for 30 s, 72 °C for 20 s with a single fluorescence measurement), melting curve program (65-97 °C with continuous fluorescence measurement), and finally a cooling step to 40 °C. The relative expression of *STA1* in the three examined yeast strains was calculated using the 'delta-delta C_{T}'-method by normalizing expression to that of the two house-keeping genes *ALG9* and *UBC6* (Pfaffl, 2001, Nucleic Acids Res., Vol 29, Issue 9, Page e45).

### Results

### Screening of diastatic ability

The diastatic ability of eighteen S. *cerevisiae* and one *S*. *pastorianus* strain (Table 1) was tested using three different tests: growth in beer, growth on starch agar, and fermentation of dextrin as a sole carbon source. Of the eighteen *S*. *cerevisiae* strains, 15 tested positive for *STA1* using the SD-5A/SD-6B primer pair (Yamauchi et al. 1998). Despite carrying the *STA1* gene, only five out of 15 strains, were able to grow in beer and on starch agar (Figure 1). These five strains also fermented dextrin efficiently. All four of the strains that tested negative for *STA1* were unable to grow in beer or on starch agar, and did not consume any dextrin.

We then amplified and sequenced the *STA1* open reading frame and a 2.5kb upstream region, to search for polymorphisms that might explain the variation in diastatic ability in the 15 strains that tested positive for *STA1.* We observed a 1162 bp deletion upstream of *STA1* (-1370 to -209 from the start codon) in 10 out of the 15 strains (Figures 1-2). This 1162 bp region contains an upstream activation sequence and transcription factor binding site (Kim et al. 2004, Mol. Cell. Biol. 24, 9542-9556; Kim et al., 2004, Mol. Cell. Biol. 24, 7695-7706). Interestingly, the presence of this deletion appeared to coincide with decreased diastatic ability (Figure 1). The strains with the deletion in the promoter exhibited significantly less diastatic ability in all tests (Figure 1A-D). We hypothesised that this deletion impedes transcription of *STA1*, which in turn decreases the amount of STA1-derived glucoamylase produced by the strains.

### Confirmation by reverse engineering

To confirm that the 1162 bp deletion that was observed in the *STA1* promoter had a negative effect on diastatic ability and *STA1* expression, we performed reverse engineering aided by the CRISPR/Cas9 system. The gRNA protospacer sequence was designed to cause a double-stranded break within the region to be deleted (Figure 2E). The 1162 bp region (-1370 to -209 upstream of the *STA1* open reading frame) was deleted from the highly diastatic WY3711 strain by transformation with a Cas9- and gRNA-expressing plasmid and an 80 bp double-stranded repair oligo. PCR using primer pairs binding within (STA1_UAS_Fw / STA1_UAS_Rv) and outside the deleted region (1055F / 2951R) was used to confirm successful deletion (Figure 2D).

The deletion strain, WY3711_D1, was then tested for diastatic ability. WY3711, the wild-type strain from which WY3711_D1 was derived, and WLP570, a strain that naturally contains the 1162 bp deletion in the promoter, were included as controls. In all tests the diastatic ability of WY3711_D1 was less than WY3711. When inoculated into beer, WY3711_D1 and WLP570 grew significantly less than WY3711 (Figure 3A). In contrast to WY3711, WY3711_D1 and WLP570 had also not consumed any dextrin from the beer after 3 weeks (Figure 3D). When the strains were grown in minimal media with dextrin as the sole carbon source, we saw negligible dextrin consumption after 3 weeks with WY3711_D1 and WLP570 when cultures were incubated anaerobically (Figure 3B). Interestingly, we observed delayed activity in these strains when cultures were incubated aerobically (Figure 3C). WY3711 performed similarly in both anaerobic and aerobic conditions, and in both cases consumed significantly more dextrin than the strains with the deletion in the promoter.

To test the effect that the 1162 bp deletion in the *STA1* promoter has on the expression of *STA1,* we performed RT-qPCR analysis on RNA extracted from exponential phase cultures in YPGE. This growth medium was chosen as glucose represses *STA1* expression (Kim et al. 2004, Mol. Cell. Biol. 24, 7695-7706; Pretorius et al., 1986, Mol. Cell. Biol. 6(9):3034-41). We observed a 150-200 fold higher abundance of *STA1* mRNA in the samples from WY3711 compared to WY3711_D1 and WLP570, respectively (Figure 3E). These results suggest that the decreased diastatic ability observed in the *STA1*+ strains with the 1162 bp deletion is a result of decreased expression of *STA1.*

### Designing primers for the detection of the full STA1 promoter

To improve the reliability of the molecular detection methods for diastatic S. *cerevisiae,* we designed two new primer pairs that bind within the 1162 bp region that is absent in the poorly diastatic *STA1*+ strains. These new primers can therefore be used to differentiate whether a strain that has tested positive for *STA1* with the SD-5A / SD-6B primers (Yamauchi et al. 1998) is likely to be highly diastatic or not. The first primer pair (STA1_UAS_Fw / STA1_UAS_Rv) was designed to produce a 599 bp amplicon, and can be used together with the SD-5A / SD-6B primers in a single multiplex PCR reaction (Figure 2B-C). Here, strains with the full *STA1* promoter produce two amplicons (599 and 868 bp), while poorly diastatic strains only produce a single amplicon (868 bp).

In addition, a second primer pair (STA1_UAS_Q_Fw / STA1_UAS_Rv) was also designed to be useable in quantitative PCR reactions where a shorter amplicon is desired. This primer pair produces a 223 bp amplicon. We performed quantitative PCR reactions on DNA extracted from *S*. *cerevisiae* WY3711, *S*. *cerevisiae* WLP570 and *S*. *pastorianus* A15 using both the previously published Sdia-f / Sdia-r primer pair that bind within the *STA1* ORF (Brandl, 2006), and our newly designed primer pair that bind within the *STA1* promoter. As expected, amplification using the Sdia-f / Sdia-r primer pair was observed for both WY3711 and WLP570 (threshold cycles of 17.0 and 18.9), while no signal above the background was observed for the negative control A15 after 40 cycles. With the new primer pair on the other hand, amplification was only observed for the highly diastatic WY3711 (threshold cycle of 18.4), while no signal above the background was observed for the poorly diastatic WLP570 and the negative control A15 after 40 cycles.

## Claims

1. A method for determining a yeast having extracellular glucoamylase STA1p activity in a sample, wherein the method comprises allowing a primer or probe capable of hybridizing with the promoter of *STA1* gene within nucleotides 1127-2288 as numbered in SEQ ID NO: 1 to hybridize with a polynucleotide of the sample and detecting the presence or absence of a deletion in the promoter of *STA1* gene of a sample, wherein the absence of the deletion in the promoter of *STA1* gene indicates the presence of extracellular glucoamylase STA1p activity of the yeast.

2. The method of claim 1, wherein the deletion comprises or is a deletion of nucleotides 1127-2288 as numbered in SEQ ID NO: 1, or the deletion is a deletion within nucleotides 1127-2288 as numbered in SEQ ID NO: 1.

3. The method of claim 1 or 2, wherein the presence or absence of the deletion is detected by a molecular method, optionally by utilizing one or more primers and/or a probe.

4. The method of claim 3, wherein the molecular method is a polymerase chain reaction (PCR), optionally a quantitative PCR, and wherein the method optionally further comprises agarose gel electrophoresis of the product obtained from the PCR.

5. The method of any preceding claim, wherein detecting the presence or absence of the deletion in the promoter of *STA1* gene is carried out by allowing a primer or probe capable of hybridizing with the promoter of *STA1* gene within nucleotides 1127-2288 as numbered in SEQ ID NO: 1 to hybridize with the DNA or RNA obtained from the sample.

6. The method of claim 5, wherein a further primer and/or probe are used for detecting STA1 and/or another gene.

7. The method of any preceding claim, wherein the presence of the deletion in the promoter of *STA1* gene indicates decreased extracellular glucoamylase STA1p activity of the yeast and/or results in decreased ethanol formation, carbon dioxide formation, hydrolysis of maltotriose and/or diastatic capability of the yeast compared to another yeast lacking the deletion in the promoter of *STA1* gene.

8. The method of any preceding claim, wherein the method further comprises detecting the presence or absence of *STA1* gene and/or optionally comprises a microbiological detection method.

9. A primer capable of hybridizing with *STA1* gene of a yeast for determining a yeast having extracellular glucoamylase STA1p activity or for detecting the presence or absence of a deletion in the promoter of STA1 gene, wherein said primer is capable of hybridizing with the promoter of *STA1* gene within nucleotides 1127-2288 as numbered in SEQ ID NO: 1, and is selected from the group consisting of a sequence as shown in SEQ ID NO: 2, a sequence as shown in SEQ ID NO: 3 and a sequence as shown in SEQ ID NO: 4.

10. A primer pair for determining a yeast having extracellular glucoamylase STA1p activity or for detecting the presence or absence of a deletion in the promoter of STA1 gene, wherein the primer pair comprises two primers capable of hybridizing with *STA1* gene of a yeast and at least one of said primers is capable of hybridizing with the promoter of *STA1* gene within nucleotides 1127-2288 as numbered in SEQ ID NO: 1, and is selected from the group consisting of a sequence as shown in SEQ ID NO: 2, a sequence as shown in SEQ ID NO: 3 and a sequence as shown in SEQ ID NO: 4.

11. The primer pair of claim 10, wherein both two primers are capable of hybridizing with the promoter of *STA1* gene within nucleotides 1127-2288 as numbered in SEQ ID NO: 1.

12. A probe capable of hybridizing with the promoter of *STA1* gene of a yeast within or at least partially within nucleotides 1127-2288 as numbered in SEQ ID NO: 1 and is selected from the group consisting of a sequence as shown in SEQ ID NO: 2, a sequence as shown in SEQ ID NO: 3 and a sequence as shown in SEQ ID NO: 4, for determining a yeast having extracellular glucoamylase STA1p activity or for detecting the presence or absence of a deletion in the promoter of STA1 gene.

13. A kit for genetic testing comprising a primer, primer pair or probe of any of claims 9-12 and optionally reagents for performing said genetic testing, and optionally wherein the kit is for the method of any of claims 1-8.

14. The primer, primer pair, probe or kit of any of the preceding claims 9-13, wherein the primer, primer pair, probe or kit is for detecting the presence or absence of a deletion in the promoter of *STA1* gene in a yeast, optionally in a sample.

15. The method of any preceding claims 1-8, wherein said primer or probe capable of hybridizing *STA1* gene of the yeast is at least 15 nucleotides, e.g. 18-50 nucleotides, 18-40 nucleotides, 18-30 nucleotides, 18-25 nucleotides, 19-24 nucleotides, 20-23 nucleotides, or 20-22 nucleotides.

16. The method of any of claims 1-8, wherein the primer capable of hybridizing with the promoter of *STA1* gene is selected from the group consisting of a sequence as shown in SEQ ID NO: 2, a sequence as shown in SEQ ID NO: 3 and a sequence as shown in SEQ ID NO: 4.

17. The method of any of claims 1-8 or 16 or the primer, primer pair, probe or kit of claim 14, wherein the sample is a food sample, biological sample, environmental sample, sample from a facility or surface, air sample or water sample, or selected from the group consisting of a product comprising a substrate for glucoamylase activity, a product of brewing industry, alcoholic beverage, beer, rice wine, sake, and soy sauce, optionally wherein the food is to be packed in a bottle, can, keg, box or package .

18. The method, primer, primer pair, probe or kit of any preceding claim, wherein the yeast belongs to a genus *Saccharomyces* or is *Saccharomyces cerevisiae.*

19. Use of the primer, primer pair, probe or kit of any preceding claim 9-14 or 17-18 for detecting the presence or absence of a deletion in the promoter of *STA1* gene in a yeast.

## Patentansprüche

1. Verfahren zum Bestimmen einer Hefe mit extrazellulärer Glucoamylase-STAlp-Aktivität in einer Probe, wobei das Verfahren einem Primer oder einer Sonde, der bzw. die in der Lage ist, mit dem Promotor des *STA1-*Gens innerhalb der Nukleotide 1127-2288 wie in SEQ ID NO: 1 nummeriert zu hybridisieren, ermöglicht, mit einem Polynukleotid der Probe zu hybridisieren, und die Anwesenheit oder Abwesenheit einer Deletion im Promotor des *STA1*-Gens einer Probe zu detektieren, wobei die Abwesenheit der Deletion im Promotor des *STA1*-Gens auf die Anwesenheit von extrazellulärer Glucoamylase-STA1p-Aktivität der Hefe hinweist.

2. Verfahren nach Anspruch 1, wobei die Deletion eine Deletion der Nukleotide 1127-2288 wie in SEQ ID NO: 1 nummeriert umfasst oder eine derartige ist, oder die Deletion eine Deletion innerhalb der Nukleotide 1127-2288 wie in SEQ ID NO: 1 nummeriert ist.

3. Verfahren nach Anspruch 1 oder 2, wobei das die Anwesenheit oder Abwesenheit der Deletion durch ein molekulares Verfahren detektiert wird, optional durch Einsetzen von einem oder mehreren Primern und/oder einer Sonde.

4. Verfahren nach Anspruch 3, wobei das molekulare Verfahren eine Polymerase-Kettenreaktion (PCR) ist, optional eine quantitative PCR, und wobei das Verfahren optional ferner eine Agarose-Gelelektrophorese des aus der PCR erhaltenen Produkts umfasst.

5. Verfahren nach irgendeinem der vorhergehenden Ansprüche, wobei das Detektieren der Anwesenheit oder Abwesenheit der Deletion im Promotor des *STA1*-Gens ausgeführt wird, indem einem Primer oder einer Sonde, der bzw. die in der Lage ist, mit dem Promotor des *STA1*-Gens innerhalb der Nukleotide 1127-2288 wie in SEQ ID NO: 1 nummeriert zu hybridisieren, ermöglicht wird, mit der aus der Probe erhaltenen DNA oder RNA zu hybridisieren.

6. Verfahren nach Anspruch 5, wobei ein weiterer Primer und/oder eine weitere Sonde zum Detektieren von STA1 und/oder einem anderen Gen verwendet werden.

7. Verfahren nach irgendeinem der vorhergehenden Ansprüche, wobei die Anwesenheit der Deletion im Promotor des STAI-Gens auf eine verringerte extrazelluläre Glucoamylase-STAlp-Aktivität der Hefe hinweist und/oder zu einer verringerten Ethanolbildung, Kohlenstoffdioxidbildung, Hydrolyse von Maltotriose und/oder diastatischen Fähigkeit der Hefe im Vergleich zu einer anderen Hefe ohne die Deletion im Promotor des STAI-Gens führt.

8. Verfahren nach irgendeinem der vorhergehenden Ansprüche, wobei das Verfahren ferner das Detektieren der Anwesenheit oder Abwesenheit des STAI-Gens umfasst und/oder optional ein mikrobiologisches Detektionsverfahren umfasst.

9. Primer, der in der Lage ist, mit dem *STA1*-Gen einer Hefe zu hybridisieren, um eine Hefe zu bestimmen, die extrazelluläre Glucoamylase-STAlp-Aktivität aufweist, oder um die Anwesenheit oder Abwesenheit einer Deletion im Promotor des STA1-Gens zu detektieren, wobei der Primer in der Lage ist, mit dem Promotor des STAI-Gens innerhalb der Nukleotide 1127-2288 wie in SEQ ID NO: 1 nummeriert zu hybridisieren, und der ausgewählt ist aus der Gruppe bestehend aus einer Sequenz wie in SEQ ID NO: 2 gezeigt, einer Sequenz wie in SEQ ID NO: 3 gezeigt und einer Sequenz wie in SEQ ID NO: 4 gezeigt.

10. Primerpaar zum Bestimmen einer Hefe mit extrazellulärer Glucoamylase-STAlp-Aktivität oder zum Detektieren der Anwesenheit oder Abwesenheit einer Deletion im Promotor des STA1-Gens, wobei das Primerpaar zwei Primer umfasst, die in der Lage sind, mit dem *STA1-*Gen einer Hefe zu hybridisieren, und zumindest einer der Primer in der Lage ist, mit dem Promotor des STAI-Gens innerhalb der Nukleotide 1127-2288 wie in SEQ ID NO: 1 nummeriert zu hybridisieren, und der ausgewählt ist aus der Gruppe bestehend aus einer Sequenz wie in SEQ ID NO: 2 gezeigt, einer Sequenz wie in SEQ ID NO: 3 gezeigt und einer Sequenz wie in SEQ ID NO: 4 gezeigt.

11. Primerpaar nach Anspruch 10, wobei beide Primer in der Lage sind, mit dem Promotor des *STA1*-Gens innerhalb der Nukleotide 1127-2288 wie in SEQ ID NO: 1 nummeriert zu hybridisieren.

12. Sonde, die in der Lage ist, mit dem Promotor des *STA1*-Gens einer Hefe innerhalb oder zumindest teilweise innerhalb der Nukleotide 1127-2288 wie in SEQ ID NO: 1 nummeriert zu hybridisieren, und die ausgewählt ist aus der Gruppe bestehend aus einer Sequenz wie in SEQ ID NO: 2 gezeigt, einer Sequenz wie in SEQ ID NO: 3 gezeigt und einer Sequenz wie in SEQ ID NO: 4 gezeigt, zum Bestimmen einer Hefe mit extrazellulärer Glucoamylase-STA1p-Aktivität oder zum Detektieren der Anwesenheit oder Abwesenheit einer Deletion in dem Promotor des STA1-Gens.

13. Kit für genetisches Testen, das einen Primer, ein Primerpaar oder eine Sonde nach irgendeinem der Ansprüche 9-12 und optional Reagenzien zum Durchführen des genetischen Testens umfasst, und wobei das Kit optional für das Verfahren nach irgendeinem der Ansprüche 1-8 ist.

14. Primer, Primerpaar, Sonde oder Kit nach irgendeinem der vorhergehenden Ansprüche 9-13, wobei der Primer, das Primerpaar, die Sonde oder das Kit zum Detektieren der Anwesenheit oder Abwesenheit einer Deletion im Promotor des *STA1*-Gens in einer Hefe, optional in einer Probe, dient.

15. Verfahren nach irgendeinem der vorhergehenden Ansprüche 1-8, wobei der Primer oder die Sonde, die in der Lage ist, mit dem *STA1*-Gen der Hefe zu hybridisieren, zumindest 15 Nukleotide, z. B. 18-50 Nukleotide, 18-40 Nukleotide, 18-30 Nukleotide, 18-25 Nukleotide, 19-24 Nukleotide, 20-23 Nukleotide oder 20-22 Nukleotide lang ist.

16. Verfahren nach irgendeinem der Ansprüche 1-8, wobei der Primer, der in der Lage ist, mit dem Promotor des *STA1*-Gens zu hybridisieren, ausgewählt ist aus der Gruppe bestehend aus einer Sequenz wie in SEQ ID NO: 2 gezeigt, einer Sequenz wie in SEQ ID NO: 3 gezeigt und einer Sequenz wie in SEQ ID NO: 4 gezeigt.

17. Verfahren nach irgendeinem der Ansprüche 1-8 oder 16 oder der Primer, das Primerpaar, die Sonde oder das Kit nach Anspruch 14, wobei die Probe eine Lebensmittelprobe, eine biologische Probe, eine Umweltprobe, eine Probe von einer Anlage oder Oberfläche, eine Luftprobe oder eine Wasserprobe ist oder ausgewählt ist aus der Gruppe bestehend aus einem Produkt, das ein Substrat für Glucoamylase-Aktivität, ein Produkt der Brauindustrie, ein alkoholisches Getränk, Bier, Reiswein, Sake und Sojasauce umfasst, wobei das Lebensmittel optional in einer Flasche, einer Dose, einem Fass, einer Schachtel oder einer Verpackung verpackt werden soll.

18. Verfahren, Primer, Primerpaar, Sonde oder Kit nach irgendeinem der vorhergehenden Ansprüche, wobei die Hefe zu einer Gattung *Saccharomyces* gehört oder *Saccharomyces cerevisiae* ist.

19. Verwendung des Primers, des Primerpaars, der Sonde oder des Kits nach irgendeinem der vorhergehenden Ansprüche 9-14 oder 17-18 zum Detektieren der Anwesenheit oder Abwesenheit einer Deletion im Promotor des STAI-Gens in einer Hefe.

## Revendications

1. Procédé pour la détermination d'une levure ayant une activité de glucoamylase STA1p extracellulaire dans un échantillon, dans lequel le procédé comprend le fait de permettre à une amorce ou à une sonde capable de s'hybrider avec le promoteur du gène *STA1* dans les nucléotides 1127 à 2288 tels que numérotés dans SEQ ID NO : 1 de s'hybrider avec un polynucléotide de l'échantillon et de détecter la présence ou l'absence d'une délétion dans le promoteur du gène *STA1* d'un échantillon, dans lequel l'absence de délétion dans le promoteur du gène *STA1* indiquant la présence d'une activité glucoamylase STA1p extracellulaire de la levure.

2. Procédé selon la revendication 1, dans lequel la délétion comprend ou est une délétion des nucléotides 1127 à 2288 tels que numérotés dans SEQ ID NO : 1, ou la délétion est une délétion dans les nucléotides 1127 à 2288 tels que numérotés dans SEQ ID NO : 1.

3. Procédé selon la revendication 1 ou 2, dans lequel la présence ou l'absence de la délétion est détectée par un procédé moléculaire, éventuellement en utilisant une ou plusieurs amorces et/ou une sonde.

4. Procédé selon la revendication 3, dans lequel le procédé moléculaire est une réaction en chaîne par polymérase (PCR), éventuellement une PCR quantitative, et dans lequel le procédé comprend éventuellement en outre une électrophorèse sur gel d'agarose du produit obtenu à partir de la PCR.

5. Procédé selon une quelconque revendication précédente, dans lequel la détection de la présence ou de l'absence de la délétion dans le promoteur du gène *STA1* est effectuée en permettant à une amorce ou à une sonde capable de s'hybrider avec le promoteur du gène *STA1* dans les nucléotides 1127 à 2288 tels que numérotés dans SEQ ID NO : 1 de s'hybrider avec l'ADN ou l'ARN obtenu à partir de l'échantillon.

6. Procédé selon la revendication 5, dans lequel une autre amorce et/ou sonde est utilisée pour détecter STA1 et/ou un autre gène.

7. Procédé selon une quelconque revendication précédente, dans lequel la présence de la délétion dans le promoteur du gène *STA1* indique une diminution de l'activité de la glucoamylase STA1p extracellulaire de la levure et/ou entraîne une diminution de la formation d'éthanol, de la formation de dioxyde de carbone, de l'hydrolyse du maltotriose et/ou de la capacité diastasique de la levure par rapport à une autre levure dépourvue de la délétion du promoteur du gène *STA1.*

8. Procédé selon une quelconque revendication précédente, dans lequel le procédé comprend en outre la détection de la présence ou de l'absence du gène *STA1* et/ou comprend éventuellement un procédé de détection microbiologique.

9. Amorce capable de s'hybrider avec le gène *STA1* d'une levure pour la détermination d'une levure ayant une activité de glucoamylase STA1p extracellulaire ou pour la détection de la présence ou de l'absence d'une délétion dans le promoteur du gène STA1, dans laquelle ladite amorce est capable de s'hybrider avec le promoteur du gène *STA1* à l'intérieur des nucléotides 1127 à 2288 tels que numérotés dans SEQ ID NO : 1, et est sélectionné dans le groupe constitué d'une séquence telle que présentée dans SEQ ID NO : 2, une séquence telle que présentée dans SEQ ID NO : 3 et une séquence telle que présentée dans SEQ ID NO : 4.

10. Paire d'amorces pour la détermination d'une levure ayant une activité de glucoamylase STA1p extracellulaire ou pour la détection de la présence ou de l'absence d'une délétion dans le promoteur du gène STA1, dans laquelle la paire d'amorces comprend deux amorces capables de s'hybrider avec le gène *STA1* d'une levure et au moins l'une desdites amorces est capable de s'hybrider avec le promoteur du gène *STA1* dans les nucléotides 1127 à 2288 tels que numérotés dans SEQ ID NO : 1, et est sélectionné dans le groupe constitué d'une séquence telle que présentée dans SEQ ID NO : 2, une séquence telle que présentée dans SEQ ID NO : 3 et une séquence telle que présentée dans SEQ ID NO : 4.

11. Paire d'amorces selon la revendication 10, dans laquelle les deux amorces sont capables de s'hybrider avec le promoteur du gène *STA1* dans les nucléotides 1127 à 2288 tels que numérotés dans SEQ ID NO : 1.

12. Sonde capable de s'hybrider avec le promoteur du gène *STA1* d'une levure à l'intérieur ou au moins partiellement à l'intérieur des nucléotides 1127 à 2288 tels que numérotés dans SEQ ID NO : 1 et est sélectionnée dans le groupe constitué d'une séquence telle que présentée dans SEQ ID NO : 2, une séquence telle que présentée dans SEQ ID NO : 3 et une séquence telle que présentée dans SEQ ID NO : 4, pour la détermination d'une levure ayant une activité de glucoamylase STA1p extracellulaire ou pour la détection de la présence ou de l'absence d'une délétion dans le promoteur du gène STA1.

13. Kit pour test génétique comprenant une amorce, une paire d'amorces ou une sonde selon l'une quelconque des revendications 9 à 12 et éventuellement des réactifs pour effectuer ledit test génétique, et éventuellement dans lequel le kit est destiné au procédé selon l'une quelconque des revendications 1 à 8.

14. Amorce, paire d'amorces, sonde ou kit selon l'une quelconque des revendications précédentes 9 à 13, dans lequel l'amorce, la paire d'amorces, la sonde ou le kit est destiné à être utilisé pour la détection de la présence ou de l'absence d'une délétion dans le promoteur du gène *STA1* dans une levure, éventuellement dans un échantillon.

15. Procédé selon l'une quelconque des revendications précédentes 1 à 8, dans lequel ladite amorce ou sonde capable d'hybrider le gène *STA1* de la levure comprend au moins 15 nucléotides, par exemple 18 à 50 nucléotides, 18 à 40 nucléotides, 18 à 30 nucléotides, 18 à 25 nucléotides, 19 à 24 nucléotides, 20 à 23 nucléotides ou 20 à 22 nucléotides.

16. Procédé selon l'une quelconque des revendications 1 à 8, dans lequel l'amorce capable de s'hybrider avec le promoteur du gène *STA1* est sélectionnée dans le groupe constitué d'une séquence telle que présentée dans SEQ ID NO : 2, une séquence telle que présentée dans SEQ ID NO : 3 et une séquence telle que présentée dans SEQ ID NO : 4.

17. Procédé selon l'une quelconque des revendications 1 à 8 ou 16, ou amorce, paire d'amorces, sonde ou kit de la revendication 14, dans lequel l'échantillon est un échantillon alimentaire, un échantillon biologique, un échantillon environnemental, un échantillon provenant d'une installation ou d'une surface, un échantillon d'air ou d'un échantillon d'eau, ou choisi dans le groupe constitué d'un produit comprenant un substrat pour l'activité de la glucoamylase, un produit de l'industrie brassicole, une boisson alcoolisée, de la bière, de l'alcool de riz, du saké et de la sauce de soja, éventuellement dans lequel l'aliment doit être conditionné dans une bouteille, une canette, un tonneau, une boîte ou un emballage.

18. Procédé, amorce, paire d'amorces, sonde ou kit selon une quelconque revendication précédente, dans lequel la levure appartient au genre *Saccharomyces* ou est *Saccharomyces cerevisiae.*

19. Utilisation de l'amorce, de la paire d'amorces, de la sonde ou du kit selon une quelconque revendication précédente 9 à 14 ou 17 et 18 pour la détection de la présence ou de l'absence d'une délétion dans le promoteur du gène *STA1* dans une levure.
